(19) Europäisches Patentamt
European Patent Office
Office européen des brevets

(11) **EP 4 173 627 A1**

(12) **EUROPEAN PATENT APPLICATION**

(43) Date of publication:
**03.05.2023  Bulletin 2023/18**

(21) Application number: **21382974.0**

(22) Date of filing: **28.10.2021**

(51) International Patent Classification (IPC):
**A61K 33/26** *(2006.01)*    **A61P 31/14** *(2006.01)*

(52) Cooperative Patent Classification (CPC):
**A61K 33/26; A61P 31/14**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Consejo Superior De Investigaciones Científicas**
**28006 Madrid (ES)**

(72) Inventors:
- **BARBER CASTAÑO, Domingo F.**
  **E - 28006 Madrid (ES)**
- **MORALES HERRERO, María del Puerto**
  **E - 28006 Madrid (ES)**
- **LÓPEZ DE DIEGO, Marta**
  **E - 28006 Madrid (ES)**
- **MULENS ARIAS, Vladimir**
  **E - 28006 Madrid (ES)**

- **PORTILLA TUNDIDOR, Yadileiny**
  **E - 28006 Madrid (ES)**
- **DAVIU BOU, Neus**
  **E - 28006 Madrid (ES)**
- **VEINTEMILLAS VERDAGUER, Sabino**
  **E - 28006 Madrid (ES)**
- **GALLO CÓRDOVA, Álvaro**
  **E - 28006 Madrid (ES)**
- **VILLAMAYOR CORONADO, Laura**
  **E - 28006 Madrid (ES)**
- **LÓPEZ GARCÍA, Darío**
  **E - 28006 Madrid (ES)**

(74) Representative: **ABG Intellectual Property Law, S.L.**
**Avenida de Burgos, 16D**
**Edificio Euromor**
**28036 Madrid (ES)**

Remarks:
The complete document including Reference Table(s) and the Sequence Listing(s) can be downloaded from the EPO website

(54) **IRON OXYHYDROXIDE NANOPARTICLES WITH ANTIVIRAL ACTIVITY AGAINST SARS AND MERS CORONAVIRUS**

(57) *Coronaviridae* viruses usually cause mild respiratory disease in humans, nevertheless, a new infectious disease caused by severe acute respiratory syndrome coronavirus 2 (SARS-CoV-2) has spread globally since December 2019, resulting in the ongoing 2019-2021 coronavirus pandemic. The present invention discloses the use of coated iron oxyhydroxide nanoparticles (IOHNPs) for use in the treatment and/or prevention of viral infections caused by *Coronaviridae,* especially those caused by respiratory syndrome-related coronaviruses such as SARS-CoV and SARS-CoV-2 and MERS-CoV. The iron oxyhydroxide nanoparticles are coated with biocompatible molecules and polymers which bind to the nanoparticle core via oxygen (e.g. sorbitol and sucrose). The IOHNPs were found stable, non-cytotoxic *in vitro* and can efficiently impair virus replication and transcription. Furthermore, the IOHNPs are suitable for oral, intranasal or parenteral administration in combination with a pharmaceutical carrier.

EP 4 173 627 A1

**Description**

**FIELD OF THE INVENTION**

The present invention relates to iron oxyhydroxide nanoparticles for use in the treatment and/or prevention of viral infections caused by *Coronaviridae,* especially those caused by respiratory syndrome-related coronaviruses selected from the species *"Severe acute respiratory syndrome-related coronavirus"* (such as SARS-CoV and SARS-CoV-2) and "*Middle East respiratory syndrome-related coronavirus*" (MERS).

**BACKGROUND**

Viruses are accountable for the common cold, influenza, AIDS, SARS, MERS, COVID-19, chikungunya, Dengue fever, zika fever, yellow fever, West Nile fever, hepatitis B, hepatitis C, hepatitis E, Ebola virus disease and rabies, among other notable human diseases. The viruses of the family *Coronaviridae* are enveloped viruses with a positive-sense single-stranded RNA genome and a nucleocapsid of helical symmetry. The name of the family is derived from the Latin corona, meaning "crown" or "halo", which refers to the characteristic appearance reminiscent of a solar corona around the virions (virus particles) when viewed under two-dimensional transmission electron microscopy, due to the surface being covered in club-shaped protein spikes.

Several members of the family *Coronaviridae* usually cause mild respiratory disease in humans (Corman et al., 2019). However, other family members such as the severe acute respiratory syndrome coronavirus (SARS-CoV) and the Middle East respiratory syndrome coronavirus (MERS-CoV) are transmitted from animals to humans and cause severe respiratory diseases in afflicted individuals, SARS and MERS, respectively (Fehr et al., 2017).

In December 2019 a new infectious disease caused by severe acute respiratory syndrome coronavirus 2 (SARS-CoV-2) was first identified in Wuhan, the capital of China's Hubei province, and has since spread globally, resulting in the ongoing 2019-2021 coronavirus pandemic. The disease has been given the name Coronavirus disease 2019 or COVID-19. COVID-19 has evolved into an appalling global pandemic worldwide.

The etiological agent of this severe respiratory syndrome was named SARS-CoV-2 due to its genetic similarity to the Severe Acute Respiratory Syndrome coronavirus (SARS-CoV) that caused the 2003 epidemic outbreak in different areas of Southeast Asia.

SARS-CoV-2 can replicate efficiently in asymptomatic patients, facilitating the spread of the virus worldwide, given that high replication levels are not necessarily associated with clinical manifestations of the disease that may restrict infected patient mobility. While silent or causing mild symptoms in a majority of cases, SARS-CoV-2 may lead to COVID-19, which is primarily but not exclusively characterized by severe pneumonia that may lead to death in a fraction of the population, most notably the elderly.

Common symptoms include fever and cough and loss of taste or smell, which may be accompanied by other such as muscle pain, diarrhoea or sore throat. While the majority of cases result in these mild symptoms, some patients progress to hypoxia, bilateral pneumonia and multi-organ failure.

COVID-19 can seriously damage the respiratory system, with some patients developing acute respiratory infection symptoms, and even acute respiratory distress syndrome (ARDS), acute respiratory failure and other complications, such as in the cardiovascular system (T. J. Guzik et al, Cardiovasc Res 2020, 116(10), 1666-1687) and central nervous system (C. ladecola et al, Cell 2020, 183(1), 16-27) which in some cases even lead to death.

Numerous efforts are being put into the development of preventive vaccines and while this is certainly the best approach to contain future outbreaks, it is clear that additional strategies need to be in place for patients that already acquired the infection.

A timely discovery of new drugs is not a viable strategy to contrast a pressing pandemic global situation, therefore the repurposing of existing drugs with established safety profile to treat COVID-19 seems more effective.

Metal and metal oxides nanoparticles have shown antiviral properties against a wide range of viruses such as influenza, HIV-1, HBV, etc.

Iron nanoparticles have considerable potential to be used as nanomedicines for different biomedical applications, including targeted drug release or magnetic resonance imaging. Previous studies have shown that the accumulation of Iron oxide nanoparticles (IONPs) inside cells induces the production of reactive oxygen species (ROS), as a consequence of IONPs degradation inside lysosomes, which trigger oxidative stress, that in turn induces the transcriptional regulation of oxidative stress-inducible genes (Portilla et al. ACS Appl. Mater. Interfaces 2021, 13(7):7924-7944). The induction of oxidative stress by IONPs might be used therapeutically in the treatment of viral infections.

In a recent report, the in vitro antiviral activity of IONPs against H1N1 influenza infection was analyzed. Results from plaque inhibition assays and quantitative real-time PCR (qPCR) of viral transcripts in the presence of IONPs suggested antiviral activity of IONPs against influenza (Kumar et al. J. Infect. Chemother. 2019, 25(5):325-329). Another report suggested that, as a consequence of their ability to induce the production of reactive oxygen species, IONPs can catalyze lipid peroxidation of the viral lipid envelope and reduce the infectivity of H1N1, H5N1, and H7N9 subtype viruses (Kim et al. Small 2017, 13(32): 1700818). In addition, it has been shown that the treatment of cells with ferric ammonium citrate (FAC) reduces influenza virus replication and production (Wang et al. Cell Discovery. 2018, 4:14).

A theoretical study (Y. Abo-Zeid et al., Eur. J. Pharm. Sci., 2020, 153, 105465) has recently shown that discrete $Fe_2O_3$ and $Fe_3O_4$ molecules can interact with the S1-RBD protein receptor domain, which is used by SARS-CoV-2 for its internalization into the host cell and subsequent replication cycling, however this modelling study does not address aspects of crucial importance such as the size or the coating of the nanoparticle. These parameters not only determine the therapeutic efficacy against the biological target but may also have an influence on the resulting toxicity of the nanoparticles. Indeed, inorganic nanoparticles have been of limited use in clinical practice due to toxicity derived from size, shape, charge and surface chemistry. For example, gold nanoparticles have been found toxic at a size of 1.4 nm but not at higher particle size (Y. Pan, Small, 2007, 3, 1941-1949).

Venofer® (Vifor Pharma Ltd), is a complex of polynuclear Fe(III) oxyhydroxide in sucrose, also known as iron-carbohydrate complex or iron sucrose, and it was developed to be administered as a replacement medication for oral iron supplements in the treatment of iron deficiency. However, no application of Venofer® has been disclosed in antiviral therapy or prophylaxis, let alone as potential treatment for severe respiratory syndrome-related coronavirus.

Therefore, specifically tailored metal oxyhydroxide nanoparticles are needed to expand and solve the limitations of current methods of treatments of *Coronaviridae* infections.

## BRIEF DESCRIPTION OF THE INVENTION

With the start of the COVID-19 pandemic, the inventors have found that iron oxyhydroxide nanoparticles (IOHNPs) display antiviral activity against SARS-CoV-2. The IOHNPs can be synthesized or, as an alternative, are commercially available and already approved by regulatory agencies as antianemic drugs (e.g. Venofer®, Yectofer®, Imferon®). Furthermore, it has been described that the severity of COVID-19 was negatively correlated with serum iron levels (Zhao et al., Open Forum Infectious Diseases, 2020, 7(7): ofaa250; Sonnweber et al., Respiratory Research. W, 2020, 21(1):276), therefore, in addition to the likely direct antiviral activity of IOHNPs, the treatment with IOHNPs could improve patient outcomes.

In addition to that, by coating the nanoparticles with a suitable organic compound, advantageous properties are displayed. Firstly, the coating confers biocompatibility to the nanoparticle which can therefore be used at a therapeutically effective concentration in living cells without causing evident cytotoxicity. Furthermore, the biocompatible coating surrounding the iron plays crucial roles in stabilizing the iron core, slowing down the release of iron, protecting the particles from further aggregation, as well as sustaining the particles in a colloidal suspension that can be intravenously injected.

Thus, a first aspect of the invention provides coated iron oxyhydroxide nanoparticles for use in the treatment or prevention of an infection caused by respiratory syndrome-related coronaviruses selected from the species "*Severe acute respiratory syndrome-related coronavirus*" and "*Middle East respiratory syndrome-related coronavirus*".

A second aspect of the invention relates to a pharmaceutical composition for use in the treatment or prevention of an infection by respiratory syndrome-related coronaviruses selected from the species *"Severe acute respiratory syndrome-related coronavirus"* and "*Middle East respiratory syndrome-related coronavirus*", said composition comprising as active ingredient an effective amount of a coated iron oxyhydroxide nanoparticle.

## BRIEF DESCRIPTION OF THE DRAWINGS

Figure 1 shows a) a TEM image, b) x-ray and c) magnetic characterization of the nanoparticles of the invention.

Figure 2 shows a) the dose-dependent cytotoxicity of Venofer® nanoparticles in Vero E6 cell lines as determined by PrestoBlue assay and b) the time-dependent IOHNPs uptake by Vero E6 cells using different doses of Venofer® (20 and 100 $\mu$g Fe/ml, where 56 $\mu$g Fe/mL = 1 mM).

Figure 3 shows the effect of therapeutic and prophylactic treatment with Venofer® nanoparticles (56 $\mu$g Fe/mL = 1mM) on SARS-CoV-2 titers in Vero E6-infected cells compared to the control experiment with untreated cells.

Figure 4 shows the therapeutic and prophylactic effects of treatment with Venofer® nanoparticles on SARS-CoV-2 replication (measured by quantifying genomic RNA, gRNA by RT-qPCR) in Vero E6-infected cells (56 $\mu$g Fe/mL = 1mM) compared to the control experiment with untreated cells.

Figure 5 shows the therapeutic and prophylactic effects of treatment with Venofer® nanoparticles on SARS-CoV-2 transcription (measured by quantifying subgenomic RNA, sgRNA by RT-qPCR) in Vero E6-infected cells (56 μg Fe/mL = 1mM) compared to the control experiment with untreated cells.

## DETAILED DESCRIPTION OF THE INVENTION

As used herein, the term "about" means a slight variation of the value specified, preferably within 10 percent of the value specified. Nevertheless, the term "about" can mean a higher tolerance of variation depending on for instance the experimental technique used. Said variations of a specified value are understood by the skilled person and are within the context of the present invention.

Unless defined otherwise, all technical and scientific terms used herein have the same meaning as commonly understood to one of ordinary skill in the art to which this disclosure belongs. As used herein, the singular forms "a", "an" and "the" include plural reference unless the context clearly dictates otherwise.

The present invention is based on the finding that administration of iron oxyhydroxide nanoparticles (IOHNPs) can be used for the treatment or prevention of "*Severe acute respiratory syndrome-related coronavirus*" and "*Middle East respiratory syndrome-related coronavirus*", specifically, it was found that the administration of iron oxyhydroxide nanoparticles in cell cultures infected by SARS-CoV-2 produced prophylactic and therapeutic effects.

The iron oxyhydroxide nanoparticles used in the present invention are surface-modified nanoparticles coated with organic molecules such as biocompatible organic molecules, surfactants, and biomacromolecules. The biocompatible coating surrounding the iron oxyhydroxide nanoparticle plays crucial roles in stabilizing the iron core, slowing down the release of iron, protecting the particles from further aggregation, as well as sustaining the particles in a colloidal suspension that can be intravenously injected. The coating may partially or completely cover the surface of the iron oxyhydroxide core to which it is bound. When the coating completely covers the iron oxyhydroxide core, a homogeneous monolayer of the biocompatible molecule is surrounding the spherical or quasi-spherical iron oxide core. Alternatively, more than one layer can cover the iron oxyhydroxide particle core by hydrophobic and/or hydrophilic interactions between the organic molecules of the coating.

As used herein, the term "iron oxyhydroxide" is used to denote a chemical compound composed of iron, oxygen and hydrogen having general formula $FeO(OH)$. The iron oxyhydroxide is either antiferromagnetic when the saturation magnetisation (Ms) is below 10 A $m^2$/kg at room temperature or weakly ferromagnetic and the iron may be as one or both of ferric iron ($Fe^{3+}$) and ferrous iron ($Fe^{2+}$).

Iron oxyhydroxides commonly form in aqueous environments with different content in iron cations ($Fe^{2+}$ and $Fe^{3+}$), oxygen, hydroxyl, water and some amounts of $SO_4^{2-}$, $CO_3^{2-}$ and $Cl^-$. Thus, iron oxyhydroxide can be in the form of hydrate $[FeO(OH){\cdot}nH_2O]$ or may also include some amounts of sulfate, carbonate and chloride ions.

Iron oxyhydroxides are also found in igneous and metamorphic rocks.

In the context of the present invention, different forms of iron oxyhydroxide can be used which differ from one another in the metal ion content and mineral structure.

Non-limiting examples of iron oxyhydroxides include ferric minerals like $Fe^{3+}OOH$ (goethite or $\alpha$-polymorph), hydrated forms of iron oxyhydroxide with different degree of hydratation, such as ferrihydrite or $(Fe^{3+})_2O_3{\bullet}0.5H_2O$, $Fe_5O_{7.5}{\cdot}4H_2O$ or $FeO(OH){\cdot}1.8H_2O$, sulphate-containing iron oxyhydroxide, such as schwertmannite or $Fe_8O_8(OH)_{5.5}(SO_4)_{1.25}$ or $Fe_8O_8(OH)_6(SO_4){\cdot}nH_2O$ or $(Fe3+)_{16}O_{16}(OH)_{9.6}(SO_4)_{3.2}{\cdot}10H_2O$, anhydrous iron oxyhydroxyde, chloride-containing iron oxyhydroxyde, such as akagaenite or $Fe_6O_5(OH)_7Cl$ or $FeO_{0.833}(OH)_{1.167}Cl_{0.167}$, and mixtures thereof.

As used herein "iron oxyhydroxide nanoparticles" (or IOHNPs) are nanoparticles containing iron oxyhydroxide and having a diameter in the nanometer scale, typically ranging between 1 nm to 500, preferably from 1 nm to 200 nm, and more preferably between 1 nm to 150 nm or even 1 nm to 100 nm.

In the context of the invention, such nanoparticles are iron oxyhydroxide nanoparticles that are coated with inorganic materials such as silica, carbon, non-metal elements, metal oxides and metal sulfides or organic molecules such as biocompatible organic molecules, surfactants, and biomacromolecules.

In a particular embodiment, the nanoparticles used in the present invention comprise a core of iron oxyhydroxide and are coated with organic molecules such as biocompatible organic molecules, surfactants, and biomacromolecules.

In a preferred embodiment, the iron oxyhydroxide is goethite, ferrihydrite or akagaenite.

In a particular embodiment, the size of the coated iron oxyhydroxide nanoparticle is comprised between 1 and 50 nm. In a preferred embodiment, the size of the coated iron oxyhydroxide nanoparticle is comprised between 2 and 20 nm. Preferably, the size of the coated iron oxyhydroxide nanoparticle is about 3 nm. The particle size can be determined by different techniques that are known in the art such as transmission electron microscopy (TEM) or, if a crystalline phase is present, X-ray diffraction (XRD). The values of particle size obtained by the techniques known in the art are in agreement within the experimental error. In the present invention, the particle size is determined by transmission electron microscopy (TEM).

In another particular embodiment, the hydrodynamic size of the coated iron oxyhydroxide nanoparticle is comprised between 25 and 100 nm, preferably between 35 and 55 nm. More preferably, the size of the coated iron oxyhydroxide nanoparticle is about 45 nm. In this context, hydrodynamic size and hydrodynamic diameter will be used interchangeably. In contrast to the particle size, the hydrodynamic size or diameter relates to the behaviour of the nanoparticle in a fluid and it represents the diameter of a hard sphere that diffuses at the same speed as the nanoparticle being measured. The hydrodynamic diameter of the coated iron oxyhydroxide nanoparticle will depend on the size of the iron oxyhydroxide core with no coating, but also on the type of coating, as well as on the type and concentration of any ions in the medium. The hydrodynamic size can be calculated by photon correlation spectroscopy.

In a particular embodiment, the coating of the iron oxyhydroxide nanoparticle is a neutrally charged organic molecule selected from the group consisting of biocompatible organic molecules, surfactants and biomacromolecules.

A "biocompatible organic molecule" is defined as an organic compound with low molecular weight, usually up to 1000 Dalton and size on the order of 1 nm that can display a biological function by binding to a biological macromolecule to alter its native activity or function. Biocompatible organic molecules may include without being limited thereto, amino acids, mono-, di- or oligo-saccharides, citric acids, succinic acids, carboxylic acids, amines, silanes, vitamins, and cyclodextrins.

A "surfactant" is defined as an organic compound that is amphiphilic, namely it contains both hydrophobic and hydrophilic groups. Example of surfactants include long fatty carbon chains with a hydrophilic head such as fatty acids, polyols, petroleum sulphonate, alkylbenzenesulphonates, naphthalene sulphonates, olefin sulphonates, alkyl sulphates, carboxylates, sulphonates, sulphated natural oils, sulphated esters, sulphated alkanolamides, ethoxylated and sulphated alkylphenols (linear or branched), ethoxylated aliphatic alcohols, polyoxyethylene surfactants, carboxylic esters, polyethylene glycol esters, anhydrosorbitol ester, glycol esters of fatty acids (oleic acid and lauric acid), carboxylic amides, monoalkanolamine condensates, polyoxyethylene fatty acid amides, quaternary ammonium salts, alkyl phosphonates such as dodecyl phosphonate, hexadecyl phosphonate, dihexadecyl phosphonate.

A "biomacromolecule" is defined as an organic compound with high molecular weight (>1000 Dalton) and a diameter ranging from about 100 to 10,000 angstroms that plays a functional role (structural, metabolic, signalling,etc.) within living cells and organisms. Examples of biomacromolecules may include natural polymers such as dextran, starch, PEGylated starch, gelatin, chitosan, proteins, polypeptides, sugars and antibodies.

In another embodiment, the above referred neutrally charged organic molecule coating the iron oxyhydroxide nanoparticle contains hydroxyl groups (-OH) that can bind to the surface of the particle core. Examples include carboxylic acids such as citric acids, cyclodextrins, monosaccharides, di-saccharides, oligo- and poly-saccharides, glycoconjugates, glycopeptides, polyethyleneglycol, dextran, carboxydextran, alginate (e.g. sodium alginate, calcium alginate, alginate sulfate). The presence of hydroxyl groups, particularly those in sugars, has a beneficial effect to the stability of iron oxyhydroxide nanoparticles additionally allowing for a narrow size distribution or polydispersity as the hydroxyl-containing ligands strongly bind to the nanoparticle surface avoiding aggregation of small particles and selectively blocking metal sites that might otherwise engage in side reactions.

In one embodiment, the neutrally charged organic molecule used for coating the iron oxyhydroxide nanoparticle has a molecular weight between 100 and 300000 Dalton, preferably between 150 and 275000 Dalton.

In a preferred embodiment, the iron oxyhydroxide nanoparticle is coated with a sugar that has a molecular weight between 180 and 300000 Dalton.

In another preferred embodiment, the neutrally charged organic molecule used for coating the iron oxyhydroxide nanoparticle is a sugar, particularly a monosaccharide, a disaccharide or a polysaccharide.

In a preferred embodiment, the sugar is a monosaccharide, even more preferably the monosaccharide is sorbitol.

In another preferred embodiment, the sugar is a disaccharide, even more preferably the disaccharide is sucrose.

In another preferred embodiment, the sugar is a polysaccharide, even more preferably the polysaccharide is dextran.

Several formulations that comprise a core of iron oxyhydroxide coated with a sugar derivative are used for the treatment of iron deficiency anemia in adult patients with chronic kidney disease. For example, Yectofer® is marketed as a formulation comprising a Fe(III)-hydroxide nanoparticle core (ferrihydrite structure) surrounded by a sorbitol coating. The particle size is tipically of about 3 nm. Yectofer® is administered intravenously and constitutes an alternative for patients that do not tolerate oral treatments for anemia. Imferon® and Dexferrum® formulations, in turn, comprise iron-dextran nanoparticles with low and high molecular weight dextran (73 and 265 KDa, respectively) and size of 4-10 nm and 30, respectively. Venofer® (Vifor Pharma Ltd) is a commercially available complex comprising polynuclear Fe(III) oxyhydroxide nanoparticles coated with sucrose. The mean particle size is about 3 nm.

In one embodiment, the iron oxyhydroxide nanoparticle comprises a particle core covered with sorbitol molecules, the coated nanoparticle having a size between 1-5 nm, preferably about 3 nm. In a preferred embodiment, the iron oxyhydroxide nanoparticles coated with sorbitol are commercially available under the brand Yectofer®.

In one embodiment, the iron oxyhydroxide nanoparticle comprises a particle core covered with sucrose molecules, the coated nanoparticle having a size between 1-5 nm and a hydrodynamic size between 40 and 50 nm.

In a preferred embodiment, the iron oxyhydroxide nanoparticles coated with sucrose are commercially available under the trademark Venofer® which are approved by regulatory agencies for use in the treatment of iron deficiency anemia in adult patients with chronic kidney disease. Venofer® consists of an iron sucrose colloidal solution. The nanoparticles in the colloidal solution comprise a core of iron oxyhydroxide, more particularly a ferrihydrite, coated with sucrose.

In one embodiment, the iron oxyhydroxide nanoparticle comprises a particle core covered with dextran, the coated nanoparticle having a size between 1 and 30 nm, preferably between 4 and 20 nm. In a preferred embodiment, the iron oxyhydroxide nanoparticles coated with dextran are commercially available under the brands Yectofer® and Dexferrum®.

Uses of the iron oxyhydroxide nanoparticles

The invention relates to the use of coated iron oxyhydroxide nanoparticles in the treatment or prevention of viral infections by RNA-viruses, preferably of the family *Coronaviridae,* especially caused by the respiratory syndrome-related coronaviruses selected from the species *"Severe acute respiratory syndrome-related coronavirus"* (such as SARS-CoV-2 and gSARS-CoV) and "*Middle East respiratory syndrome-related coronavirus*" (MERS-CoV).

The invention also relates to a method of treating or preventing viral infections by RNA-viruses, preferably of the family *Coronaviridae,* especially caused by the respiratory syndrome-related coronaviruses selected from the species "*Severe acute respiratory syndrome-related coronavirus*" (such as SARS-CoV-2 and SARS-CoV) and "*Middle East respiratory syndrome-related coronavirus*" (MERS-CoV) in a subject, comprising administering to said subject a therapeutically effective amount of coated iron oxyhydroxide nanoparticles.

The term "viruses of the family *Coronaviridae*", as used herein, is used to designate any viral species of the taxonomic family "*Coronaviridae*" including those of the genera "*Alphacoronavirus*", "*Betacoronavirus*", "*Gammacoronavirus*" and "*Deltacoronavirus*".

In a preferred embodiment of the present invention, the viruses are selected from betacoronaviruses, in particular from the lineage "*Sarbecovirus*" and still more preferably from the species *"Severe acute respiratory syndrome-related coronavirus"* (such as SARS-CoV and SARS-CoV-2) and "*Middle East respiratory syndrome-related coronavirus*" (MERS-CoV).

In a more preferred embodiment, the viral infection is an infection caused by the species *"Severe acute respiratory syndrome-related coronavirus"*. In a particular embodiment, the viral infection is an infection by SARS-CoV-2 virus.

The terms "treating", "treatment", and therapeutic treatment, as used herein, means reversing, alleviating, inhibiting the progress of, the disease or condition to which such term applies, or one or more symptoms of such disease or condition, such as lowering the viral load in a patient with respect to pre-treatment levels.

The terms "preventing", "prevention", and "prophylactic treatment", as used herein, means avoiding or inhibiting the onset of one or more symptoms of coronavirus infections such as fever, cough, shortness of breath, muscle pain, sputum production, diarrhea, sore throat, loss of smell, pneumonia and abdominal pain.

The terms "SARS-CoV-2 titers" or "viral titers" as used herein, refers to the concentration of infectious viral particles as measured in the extracellular media, thus a reduction of SARS-CoV-2 titers means a reduction of the concentration of infectious SARS-CoV-2 viral particles compared to a control in untreated cells.

Preferably, the IOHNPs disclosed herein, are used for the treatment and/or prevention of viral infections by *"Severe acute respiratory syndrome-related coronavirus"* and most preferably for the treatment and/or prevention of viral infections by SARS-CoV-2.

In another particular embodiment, the viral infection is an infection by "*Severe acute respiratory syndrome-related coronavirus*" and most preferably by SARS-CoV-2.

The treatment of cells infected by SARS-CoV-2 with IOHNPs reduced viral titers, when cells were treated with IOHNPs before (prophylactic treatment) or after (therapeutic treatment) SARS-CoV-2 infection. In preferred embodiments, the prophylactic treatment of cells with IOHNPs reduces SARS-CoV-2 titers by at least 70%, preferably by 80%.

In one embodiment, the prophylactic treatment of cells infected by SARS-CoV-2 with iron oxyhydroxide nanoparticles covered by sucrose reduces SARS-CoV-2 titers by at least 60%, preferably by at least 80%.

In one embodiment, the therapeutic treatment of cells infected by SARS-CoV-2 with iron oxyhydroxide nanoparticles covered by sucrose reduces SARS-CoV-2 titers by at least 60%, preferably by at least 80%.

In one embodiment, the prophylactic treatment of cells infected by SARS-CoV-2 Venofer® reduces SARS-CoV-2 titers by at least 60%, preferably by at least 80%.

In one embodiment, the therapeutic treatment of cells infected by SARS-CoV-2 Venofer® reduces SARS-CoV-2 titers by at least 60%, preferably by at least 80%.

Additionally, the treatment of cells infected by SARS-CoV-2 with IOHNPs directly impaired SARS-CoV-2 virus replication and/or transcription, when cells were treated with IOHNPs before (prophylactic treatment) or after (therapeutic treatment) SARS-CoV-2 infection. The amount of genomic RNA (gRNA), which determines virus replication, and gene 7 subgenomic messenger RNA (sg mRNA), which determines virus transcription, can be determined by Real-Time Quantitative Reverse Transcription PCR (qRT-PCR). In some embodiments, the treatment of cells before or after SARS-CoV-2 infection with coated iron oxyhydroxide nanoparticles decreases the amounts of gRNA and gene 7 sg mRNA in both therapeutic and prophylactic treatments.

In a particular embodiment, the prophylactic treatment of cells infected by SARS-CoV-2 with iron oxyhydroxide nanoparticles covered by sucrose reduces the amount of viral gRNA by at least 60%, preferably by at least 80%.

In a particular embodiment, the therapeutic treatment of cells infected by SARS-CoV-2 with iron oxyhydroxide nanoparticles covered by sucrose reduces the amount of viral gRNA by at least 50%.

In a particular embodiment, the prophylactic treatment of cells infected by SARS-CoV-2 with iron oxyhydroxide nanoparticles covered by sucrose reduces the amount of viral gene 7 sg mRNA by at least 60%, preferably by at least 75%.

In a particular embodiment, the therapeutic treatment of cells infected by SARS-CoV-2 with iron oxyhydroxide nanoparticles covered by sucrose reduces the amount of viral gene 7 sg mRNA by at least 20%, preferably by at least 30%.

In a particular embodiment, the prophylactic treatment of cells infected by SARS-CoV-2 with Venofer® reduces the amount of viral gRNA by at least 60%, preferably by at least 80%.

In a particular embodiment, the therapeutic treatment of cells infected by SARS-CoV-2 with Venofer® reduces the amount of viral gRNA by at least 50%.

In a particular embodiment, the prophylactic treatment of cells infected by SARS-CoV-2 with Venofer® reduces the amount of viral gene 7 sg mRNA by at least 60%, preferably by at least 75%.

In a particular embodiment, the therapeutic treatment of cells infected by SARS-CoV-2 with Venofer® reduces the amount of viral gene 7 sg mRNA by at least 20%, preferably by at least 30%.

The nanoparticles for use according to the invention may be administered by any appropriate route (via), such as, oral (e.g., oral, sublingual, etc.) or parenteral (e.g., subcutaneous, intramuscular, intravenous, etc.). The nanoparticles for use according to the invention may also be administered intranasally, preferably in the form of an aerosol.

In particular, the iron oxyhydroxide nanoparticles for use according to the invention are administered as a pharmaceutical composition, which comprises said coated nanoparticles and one or more pharmaceutically acceptable excipients.

The coated iron oxyhydroxide nanoparticles can be used in a pharmaceutically acceptable composition that comprises the coated nanoparticles embedded within a carrier, such as hydrogels, liposomes or shell coating. Other typical carriers are known to the person skilled in the art.

In another embodiment, the pharmaceutical composition comprising the coated nanoparticles embedded within a carrier, such as hydrogels, liposomes or shell coating further comprises one or more pharmaceutically acceptable excipients.

In an embodiment, the nanoparticles for use according to the invention are administered in combination with one or more pharmaceutically acceptable excipients via oral, intranasal or parenteral administration.

In a preferred embodiment, the coated iron oxide nanoparticle for use according to the invention are administered in combination with one or more pharmaceutically acceptable excipients by intravenous (i.v), intracoronary (i.c), intramuscular (i.m), intraperitoneal (i.p), or subcutaneous (s.c.) injection.

In a particular embodiment, the coated iron oxide nanoparticles for use according to the invention are administered intranasally, preferably in the form of an aerosol.

The term "pharmaceutically acceptable excipient" refers to an inert, non-toxic vehicle, diluent, or adjuvant is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similar. Water or saline aqueous solutions and aqueous dextrose and glycerol solutions, particularly for injectable solutions, are preferably used as vehicles. Suitable pharmaceutical vehicles are known by the skilled person. The pharmaceutically acceptable excipient necessary to manufacture the desired pharmaceutical composition of the invention will depend, among other factors, on the elected administration route. Said pharmaceutical compositions may be manufactured according to conventional methods known by the skilled person in the art.

The compounds for use according to the invention may be administered in a "therapeutically effective amount", i.e. a non-toxic but sufficient amount of the corresponding compound to provide the desired effect. The amount that is "effective" will vary from subject to subject, depending on the age and general condition of the individual, the particular compound administered, and the like. However, an appropriate amount in any individual case may be determined by one of ordinary skill in the art using routine experimentation.

The iron oxyhydroxide nanoparticles are administered and dosed taking into account the clinical condition of the individual, the site and method of administration, scheduling of administration, patient age, sex, body weight and other factors known to medical practitioners. The nanoparticles for use according to the invention will typically be administered once or more times a day, for example 1, 2, 3 or 4 times daily over a period of several days, with typical total daily doses depending on the particular compound and severity of the disease, and may be easily determined by the skilled practitioner.

By way of example, typical total doses for the nanoparticles of the invention are in the range of 0.5-25 mg/Kg body weight, preferably in the range of 1-10 mg/Kg body weight, even more preferably in the range of 1.5-5 mg/Kg body weight, when administered as single dose or up to 5 consecutive doses on alternate days.

The pharmaceutical compositions may be prepared using standard methods such as those described or referred to in the Spanish, European and US Pharmacopoeias and similar reference texts.

The term "subject" refers to a mammal, e.g., a human.

The nanoparticles for use according to the invention may be administered as the sole active ingredient or in combination with other active ingredients. In a particular embodiment, the nanoparticles are used as the sole active ingredient. In another particular embodiment, the nanoparticles are used in combination with other active ingredients such as one or more anti-inflammatory agents. Examples of anti-inflammatory agents which may be combined with the iron oxyhydroxide particles include, without being limited thereto, steroidal anti-inflammatory drugs and non-steroidal anti-inflammatory drugs (NSATD). The additional agent may be administered to the subject before, concomitant or after administration of the iron oxyhydroxide nanoparticles. When administered together with the iron oxyhydroxide particles, the two (or more) may be in the same formulation or formulated in two different formulations.

In a particular embodiment, the nanoparticles of the invention are used in combination with one or more compounds useful in the treatment of viral infections by RNA-viruses, preferably of the family *Coronaviridae,* especially by the respiratory syndrome-related coronaviruses selected from the species "*Severe acute respiratory syndrome-related coronavirus*" (such as SARS-CoV-2 and SARS-CoV) and "*Middle East respiratory syndrome-related coronavirus*" (MERS-CoV). Some compounds that have been disclosed as potentially useful are selected from the group consisting of clofazimine, indomethacine, invermectin, disulfiram, boceprevir, paritaprevir, telaprevir, simeprevir, remdesivir, ribavirin, favipiravir, chloroquine, hydroxychloroquine, lopinavir, ritonavir, umifenovir and recombinant type I interferon, preferably clofazimine, indomethacine, invermectin, disulfiram, boceprevir, paritaprevir, telaprevir, simeprevir, remdesivir, lopinavir and ritonavir. Preferably, the combination is synergistic.

The term "combination" refers to a product comprising the defined nanoparticles and one or more of the compounds abovementioned potentially useful for treating respiratory syndrome-related coronaviruses, either in a single composition or in several compositions (or units), in which case the corresponding nanoparticles are distributed among the several compositions. Preferably, the combination refers to several compositions, in particular comprising one composition (or unit) per compound (compound as defined above) of the combination. The expression "one or more" when characterizing the combination refers to at least one, preferably 1, 2, 3, 4, or 5 compounds, more preferably, 1, 2 or 3 compounds, even more preferably 1 or 2 compounds.

When the combination is in the form of a single composition, the nanoparticles and the compounds present in the combination are always administered simultaneously.

When the combination is in the form of several compositions (or units), each of them having at least one of the compounds of the combination, the compositions or (units) may be administered simultaneously, sequentially or separately.

Simultaneous administration means that the nanoparticles, compounds or compositions (or units) are administered at the same time.

Sequential administration means that the nanoparticles, compounds or compositions (or units) are administered at different time points in a chronologically staggered manner.

Separate administration means that the nanoparticles, compounds or compositions (or units) are administered at different time points independently of each other.

In particular, the combinations for use according to the invention are administered as pharmaceutical compositions, which comprise the corresponding nanoparticles and (active) compounds and a pharmaceutically acceptable excipient, as previously defined.

The combinations for use according to the invention will typically be administered once or more times a day, for example 1, 2, 3 or 4 times daily, with typical total daily doses depending on the particular compound and severity of the disease, and may be easily determined by the skilled practitioner.

In this context, the present disclosure thus also provides a method of treatment or prevention of an infection by respiratory syndrome-related coronaviruses selected from the species *"Severe acute respiratory syndrome-related coronavirus"* and "*Middle East respiratory syndrome-related coronavirus*" comprising administering to a subject in need of treatment of said infection of an amount of iron oxyhydroxide nanoparticles, the amount being effective to treat the infectious disease.

Further, in this context, the present disclosure provides the use of iron oxyhydroxide nanoparticles for the preparation of a medicament for the treatment or prevention of an infection by respiratory syndrome-related coronaviruses selected from the species *"Severe acute respiratory syndrome-related coronavirus"* and "*Middle East respiratory syndrome-related coronavirus*".

The following examples represent specific embodiments of the present invention. They do not intend to limit in any way the scope of the invention defined in the present description.

**EXAMPLES**

Characterization

The following methods were employed in the different analyses mentioned in the following examples. Particle size and shape were studied by transmission electron microscopy using a 100 keV JEOL microscope. TEM samples were prepared by placing one drop of a dilute suspension of nanoparticles in water on a carbon coated copper grid and allowing the solvent to evaporate slowly at room temperature. The mean particle size and distribution were evaluated by measuring at least 250 particles.

The phase of the iron oxyhydroxide particles was identified by powder X-ray diffraction and was found in agreement with literature data (L. Gutiérrez et al., Journal of Magnetism and Magnetic Materials 293(1):69-74, 2005). The X-ray patterns were collected between 10° and 80° ($2\theta$) in a Bruker D8 Advance diffractometer with Cu K$\alpha$ radiation. The x-ray diffraction for Venofer® consists of nanometric particles most likely composed of two-line ferrihydrite. The particles are poorly crystallized nanoparticles as they are synthesized in water at low temperature. The crystal size (DXRD) was calculated from the broadening of the (311) reflection of the spinel structure following standard procedures and was in agreement with the size derived from TEM.

The presence of adsorbed anions on the particle surface was also investigated by thermogravimetric (TG) analysis of the powders in a Seiko TG/ATD 320 U, SSC 5200. The analysis was performed at room temperature up to 900 °C at a heating rate of 10 °C min$^{-1}$ in an air flow.

Colloidal properties of the samples were studied in a Zetasizer Nano S, from Malvern Instruments. The hydrodynamic size of the particles in suspensions was measured by photon correlation spectroscopy and the electrophoretic mobility was measured as a function of pH at 25 °C, using $10^{-2}$M $KNO_3$ as electrolyte and $HNO_3$ and $KOH$ to change the pH of the suspensions. The hydrodynamic size of the Venofer® particles is about 45 nm.

Magnetic characterization of the samples was carried out in a vibrating sample magnetometer (MLVSM9 MagLab 9 T, Oxford Instruments) at room temperature. Magnetization curves were recorded by first saturating the sample in a field of 5 T; then, the saturation magnetization (Ms), and the coercive field (Hc) were determined for each sample. Ms values were evaluated by extrapolating to infinite field the experimental results obtained in the high field range where magnetization linearly increases with 1/H. Samples were measured in powder form, pressed in a pellet, and were found to be composed of antiferromagnetic nanoparticles.

Electrophoretic mobility data were transformed to zeta potential values, which are related to the surface charge density and depend on the coating.

Iron leaching was analysed by diluting in a buffer (pH 5) the nanoparticles, which were kept at 37°C for 24 h and then separated using Amicon® Ultra Centrifugal filters. The iron content in the supernatant was determined by inductively coupled plasma - optical emission spectrometry (ICP-OES).

Cell lines, viruses and reagents

The African green monkey kidney-derived epithelial Vero E6 cells were kindly provided Centro Nacional de Biotecnología-CSIC. Vero E6 cells were grown in Dulbecco's modified Eagle's medium (DMEM) (Gibco, Invitrogen, CA) supplemented with 25 mM HEPES and 10% fetal bovine serum (Fisher).

SARS-CoV-2, isolated in Vero E6 cells, originating from a nasal swab from a patient infected in Madrid (Spain), was also kindly provided by Centro Nacional de Biotecnología-CSIC.

Example 1. Characterization iron oxyhydroxide nanoparticles (IOHNPs)

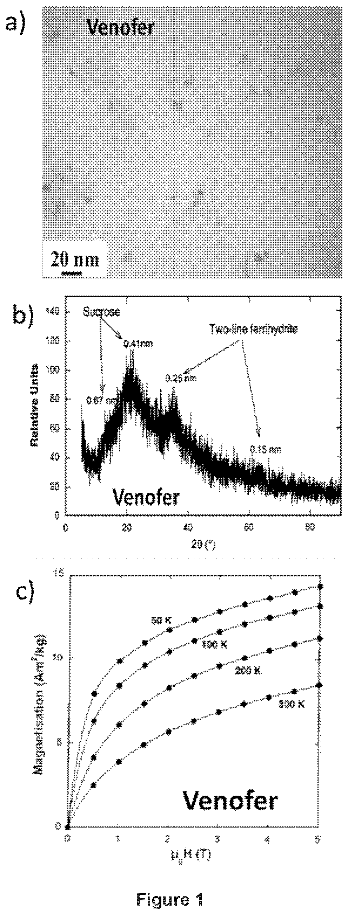

Venofer® (Vifor Pharma Ltd) is a commercially available complex of polynuclear Fe(III) oxyhydroxide with sucrose. The nanoparticles were characterized by TEM, XRD, thermogravimetric analysis, photon correlation spectroscopy and vibrating-sample magnetometry. Figure 1 shows TEM images of a Venofer sample as well as the x-ray diffractogram and magnetic saturation curve. Table 1 summarizes the main structural and physiochemical features of the iron oxyhydroxide nanoparticles.

Dissolution of the nanoparticles was evaluated by analysing the iron leaching after 24 h at 37 °C, and it was 1.75 %, is similar to other magnetic iron oxide nanoparticles based on magnetite or maghemite cores of the same size but above larger size particles (0.6-0.2%).

Table 1. Characterization of Venofer® nanoparticles.

| Sample | Coating | Particle size TEM (nm) | Crystal size X-ray (nm) | Hydrodyna mic size (nm) | Z-Potential (mV) | Ms (A m²/Kg$_{Fe}$) |
|---|---|---|---|---|---|---|
| Venofer® | Sucrose | 3 | 3.8 | 45 (PDI=0.21) | -10 | 14 |
| PDI: polydispersity | | | | | | |

Example 2. Biological assays

Cytotoxicity assays by PrestoBlue

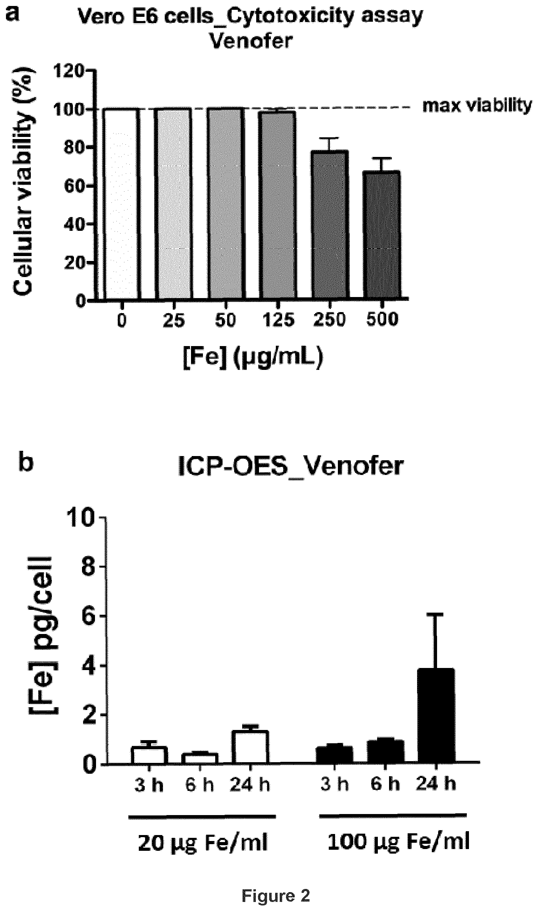

Cell viability was determined with the colorimetric Presto Blue assay (Invitrogen). Presto Blue reagent contains a cell permeable blue and non-fluorescent solution of resazurin, which can be metabolized inside cells to a red and fluorescent compound called resorufin. The change of color and fluorescence serves as a cell viability indicator. Vero E6 cells were seeded in a 96-well plate at a density of $3\times10^4$ cells in a total volume of $100\,\mu l$ per well. After 24h incubation at 37°C, Vero E6 cells were incubated with different concentrations of Venofer® nanoparticles suspensions (0-500 $\mu$g Fe/ml) for 24h. After the nanoparticles incubation, PrestoBlue reagent was added to each well, incubated in the same culture conditions for 2h and fluorescence was measured (560nm excitation; 590nm emission). Cell viability (see Figure 2a) is indicated as percentage of fluorescence of treated cells compared to fluorescence of the non-treated cells following

$$Cell\ Viability\ (\%) = \frac{fluorescence\ treated\ cells - medium\ fluorescence}{fluorescence\ non\ treated\ cells - medium\ fluorescence} \times 100$$

the equation:

As it appears in figure 2a, Venofer® nanoparticles did not display detectable cytotoxicity in Vero E6 cells up to a concentration of 125 $\mu$g Fe/mL (about 2.5 mM), while at 250 $\mu$g Fe/mL (about 5 mM) the cell viability decreased to about 80%. More evident cytotoxicity was observed at higher concentrations (500 $\mu$g Fe/mL, that is about 9 mM), therefore the concentration for use in further in vitro studies was up to 250 $\mu$g Fe/mL (about 5 mM).

Quantification of iron uptake by ICP-OES

Vero E6 cells were seeded in a 6-well plate at a density of $1\times10^5$ cells per well and cultured for 24 h at 37 °C. After this, the Vero E6 cells were incubated with Venofer® nanoparticles (20 or 100 $\mu$g Fe/mL, that is about 0.4 or 2 mM, respectively) in the same culture conditions for 3, 6 or 24 h. Subsequently, the cells were then washed three times with phosphate-buffered saline (PBS) to remove non-internalized nanoparticles, harvested and counted in a Neubauer chamber. The samples were digested in $HNO_3$ (1 mL) and $H_2O_2$ (1 mL) for 1h at 90 °C, the amount of IOHNP internalized by Vero E6 cells or amount of iron per cell at 20 $\mu$g Fe/ml (about 0.4 mM) and 100 $\mu$g Fe/ml (about 2 mM) and different incubation times of IOHNPs (see Figure 2b) was determined by ICP-OES (inductively coupled plasma - optical emission spectrometry) with a Perkin Elmer-2400 instrument. More specifically, the amount of IOHNP internalized by Vero E6 cells at 24h was about 1.3 and 3.8 pg iron/cell at concentrations of 20 or 100$\mu$g Fe/mL (about 0.4 or 2 mM), respectively.

Virus infections

To analyze the prophylactic effect of the iron nanoparticles, confluent monolayers of Vero E6 cells (24-well-format plates) were treated for 24 h with Venofer® nanoparticles, at 20 and 100 $\mu$g Fe/ml. The studied concentrations were found non-cytotoxic in previous studies (see "Cytotoxicity assays by PrestoBlue" section). The cells were then infected with SARS-CoV-2 (multiplicity of infection, MOI, 0.001), for 24 and 48 h. Cell culture media were collected and titrated 24 and 48 hours post infection (hpi). In addition, at 6 and 16 hpi, cells were collected and used for total RNA purification.

In a different experiment, the therapeutic effect of the nanoparticles was evaluated by infecting confluent monolayers of Vero E6 cells (24-well-format plates) with SARS-CoV-2 (MOI, 0.001). Venofer® nanoparticles were then added to the media at 1 hpi, using the same concentrations as those employed to evaluate the prophylactic effect. Extracellular media were collected and titrated 24 and 48 hpi. In addition, at 6 and 16 hpi, cells were collected and used for total RNA purification.

Virus titrations

SARS-CoV-2 virus titrations were performed in Vero E6 cells grown in 24-well plates and infected with 10-fold serial dilutions of the virus. After 1h absorption, cells were overlaid with low electroendosmosis agarose (Pronadisa) and incubated for 3 days at 37°C. For all virus titrations, cells were fixed with 10% formaldehyde in phosphate buffer saline (PBS) and permeabilized with 20% methanol. Viral plaques were visualized and counted using crystal violet.

Analysis of virus replication and transcription

Total RNA from untreated or Venofer®-treated cells (either mock-infected or SARS-CoV-2-infected) were extracted using a total RNA extraction kit (Omega Bio-tek, GA, USA) following the manufacturer's recommendations. Purified RNA (1 μg) was reverse-transcribed to cDNA using a high-capacity cDNA reverse-transcription kit (Applied Biosystems) and random primers.

To analyze the effect of the treatments with nanoparticles on virus replication, qPCRs using the primers SARS-2-RdRp-15431-VS (5'GTGAAATGGTCATGTGTGGCGG-3') and SARS-2-RdRp-15530-RS (5'- CAAATGTTAAAAACACTATTAGCATA-3'), complementary to the viral genomic RNA (gRNA) were performed according to the procedure in T. Toptan et al., Int. J. Mol. Sci. 2020, 21(12), 4396).

To analyze the effect of the NPs on virus transcription, the primers, SARS-2-leader-VS: 5'-TCCCAGGTAACAAACCAACCAACT, complementary to the leader sequence; and SARS-2-7a-RS: 5'-AAATGGTGAATTGCCCTCGT-3, complementary to gene 7 open reading frame, were used, to amplify the gene 7 subgenomic messenger RNA (sg mRNA) by qPCR according to the procedure in Alexandersen et al., Nature Communications, 2020, 6059. In all the cases, GAPDH was used to normalize the data using the 5'-TGCCATGGGTGGAATCATATTGGA-3' (sense) and 5'-TCGGAGTCAACGGATTTGGGTCGT-3 (antisense) primers. Quantification was achieved using the threshold cycle (2-ΔΔCT) method (K. J. Livak et al, Methods, 2001, 25(4), 402-408).

Analysis of the effect of oxidative stress on the antiviral activity of Venofer®

Confluent monolayers of Vero E6 cells (24-well-format plates) were treated during 24 h with N-acetylcysteine (NAC), recognized as a reactive oxygen species scavenger, at a 200 uM concentration or left untreated, as control. Then, the cells were infected with SARS-CoV-2 (MOI, 0.001), and at 1 hpi, the extracellular medium containing the virus was replaced with a suspension of Venofer nanoparticles and NAC solution (200 uM) or with containing suspension of the nanoparticles without NAC, as control. Media were collected and titrated at 48 hpi.

Effect of nanoparticles on viral production

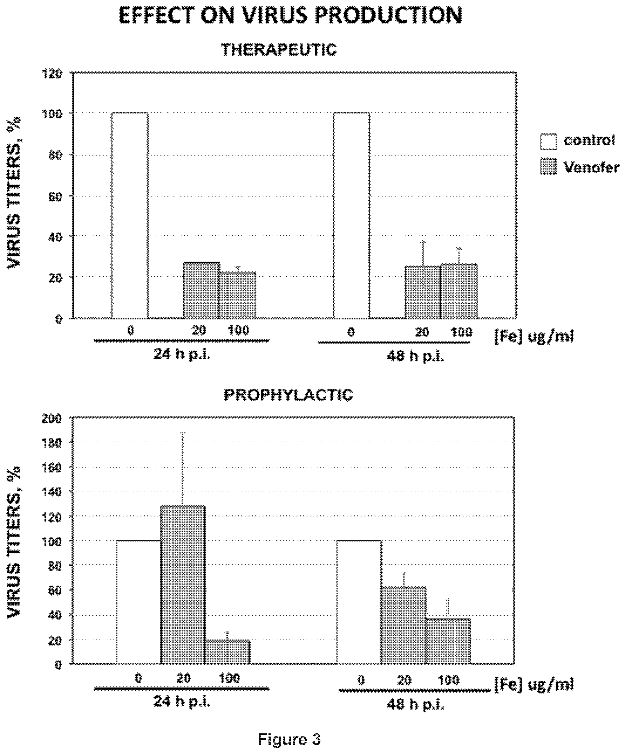

In order to verify whether Venofer® nanoparticles affect SARS-CoV-2 production, cells were either treated with nanoparticles, 24 h before infection (prophylactic effect) or 1 h after infection (therapeutic effect), and virus titers at different times post-infection were measured (see "Virus Titrations" section above). Interestingly, for both the therapeutic and prophylactic effect, a dose-dependent effect of nanoparticles on reducing infectious viruses produced by infected cells was observed (see Figure 3).

For the therapeutic treatment, the highest, non-cytotoxic concentration of the nanoparticles decreased SARS-CoV-2 production at both 24 and 48 hpi, in fact, at a concentration of 100 μg Fe/ml (about 2 mM), Venofer nanoparticles reduced virus titers by 78% at 24 hpi, and by 75 % at 48 hpi (Figure 3).

For the prophylactic treatment, the highest concentration of Venofer® nanoparticles also reduced virus titers at 24 and 48 hpi. The maximum reduction of virus titers in cells which were first treated with the nanoparticles and then infected with SARS-CoV-2 was observed using the 100 μg Fe/mL concentration of Venofer® nanoparticles (about 2 mM), which reduced viral titers by 80% and 62% at 24 and 48 hpi, respectively (Figure 3). These results indicated that the treatment of cells with Venofer® nanoparticles substantially reduced the production of SARS-CoV-2 infectious viruses compared to the untreated cells.

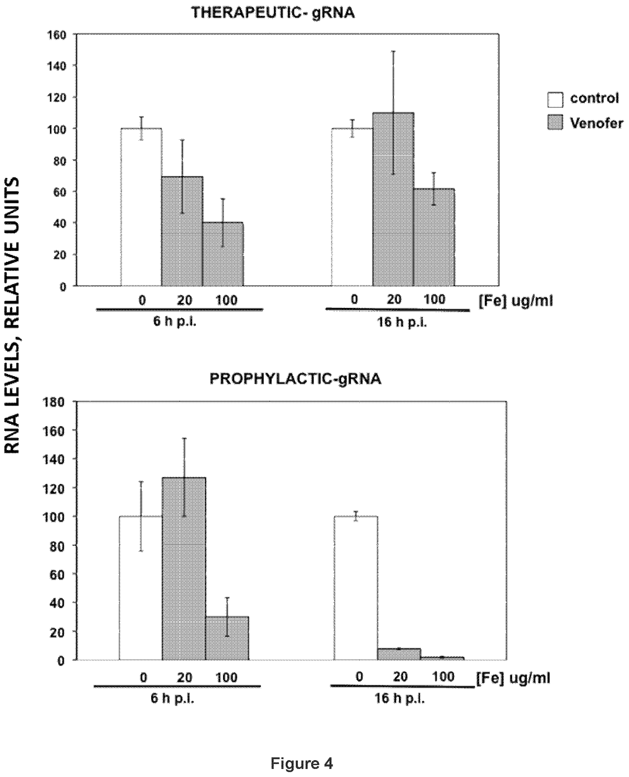

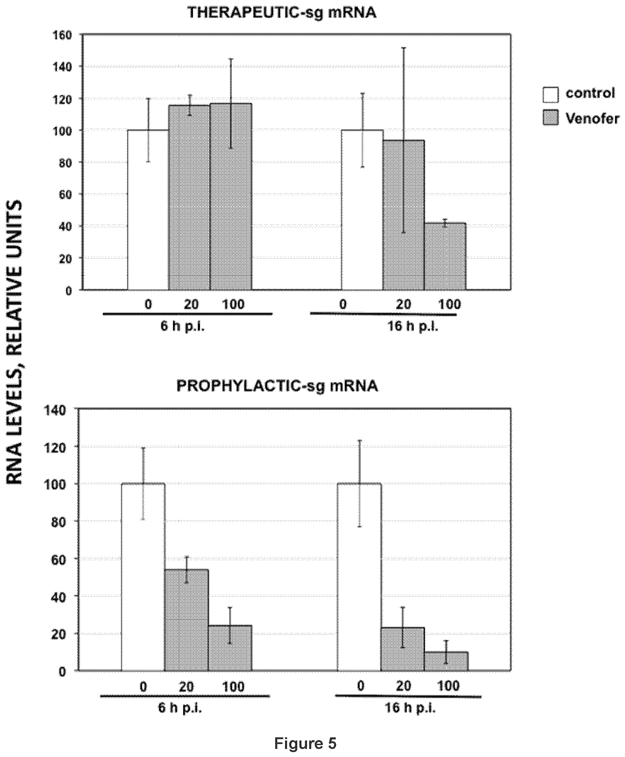

In order to gain insight on whether the treatment of cells with IOHNPs directly affects virus replication and/or transcription, cells were treated with Venofer® nanoparticles before or after SARS-CoV-2 infection. The amounts of genomic RNA, which determines virus replication, and sg mRNA, which determines virus transcription, were measured by qRT-PCR at 6 and 16 hpi. Remarkably, the treatment of cells before or after SARS-CoV-2 infection with the highest dosis of Venofer® nanoparticles decreased the amounts of gRNA and gene 7 sg mRNA at 16 hpi in both therapeutic and prophylactic treatments (see figures 4 and 5). These results strongly suggested that the treatment of cells with Venofer® nanoparticles impaired virus replication and transcription.

To verify whether the oxidative stress induced by the Venofer® nanoparticles is responsible for their antiviral effect (Mulens-Arias et al., Biomaterials, 2015, 52:494-506; Khan et al., Biomaterials, 2012, 33(5):1477-88; Portilla et al. ACS Appl. Mater. Interfaces, 2021, 13(7):7924-7944), cells were treated with the above nanoparticles and NAC, a ROS (reactive oxygen species) scavenger, and viral titers were measured at 48 hpi. Interestingly, viral titers were similar in cells treated with NAC and in control cells where NAC was not applied, suggesting that oxidative stress does not affect the antiviral activity of Venofer®.

**Claims**

1. A coated iron oxyhydroxide nanoparticle for use in the treatment or prevention of an infection caused by respiratory syndrome-related coronaviruses selected from the species Severe acute respiratory syndrome-related coronavirus and Middle East respiratory syndrome-related coronavirus.

2. The coated iron oxyhydroxide nanoparticle for use according to claim 1, wherein the respiratory syndrome-related coronavirus is selected from the group consisting of SARS-CoV-2, SARS-CoV and MERS-CoV, preferably SARS-CoV-2.

3. The coated iron oxyhydroxide nanoparticle for use according to claims 1 and 2, wherein the size of the coated nanoparticle is comprised between 1 and 50 nm, preferably between 2 and 20 nm.

4. The coated iron oxyhydroxide nanoparticle for use according to claims 1 and 3, wherein the iron oxyhydroxide is goethite, ferrihydrite or akagaenite.

5. The coated iron oxyhydroxide nanoparticle for use according to claims 1 to 4, wherein the nanoparticle is coated with inorganic materials selected from silica, carbon, non-metal elementary substance, metal oxides and metal sulphides; or with a neutrally charged organic molecule selected from the group consisting of biocompatible organic molecules, surfactants, and biomacromolecules.

6. The coated iron oxyhydroxide nanoparticle for use according to claims 1 to 5, wherein the nanoparticle is coated with a neutrally charged organic molecule that contains hydroxyl groups.

7. The coated iron oxide nanoparticle for use according to claim 5 or 6, wherein the neutrally charged organic molecule used for coating has a molecular weight between 100 and 300000 Dalton, preferably between 150 and 275000 Dalton.

8. The coated iron oxyhydroxide nanoparticle for use according to claims 1 to 7, wherein the nanoparticle is coated with a sugar molecule selected from a monosaccharide, a di-saccharide and a poly-saccharide.

9. The coated iron oxyhydroxide nanoparticle for use according to claim 8, wherein the disaccharide is sucrose.

10. The coated iron oxyhydroxide nanoparticle for use according to claims 1 to 8, wherein the size of the coated iron nanoparticle is comprised between 2 nm and 20 nm and the iron oxyhydroxide core is coated with sucrose.

11. The coated iron oxide nanoparticle for use according to claims 1 to 10, wherein the coated nanoparticles are commercial Venofer® nanoparticles.

12. The coated iron oxyhydroxide nanoparticle for use according to claim 1 to 11, wherein the hydrodynamic diameter of the nanoparticles is comprised between 25 and 100 nm, more preferably between 35 and 55 nm.

13. The coated iron oxyhydroxide nanoparticle for use according to claims 1 to 10, alone or in combination with a pharmaceutical carrier, for oral, intranasal or parenteral administration.

14. The coated iron oxyhydroxide nanoparticle for use according to claim 13, wherein the parenteral administration is performed by intravenous (i.v), intracoronary (i.c), intramuscular (i.m), intraperitoneal (i.p) or subcutaneous (s.c.) injection.

15. The coated iron oxide nanoparticle for use according to claim 13, wherein the intranasal administration is performed by aerosols.

Figure 1

Figure 2

EFFECT ON VIRUS PRODUCTION

Figure 3

Figure 4

Figure 5

Europäisches
Patentamt

European
Patent Office

Office européen
des brevets

## EUROPEAN SEARCH REPORT

Application Number

EP 21 38 2974

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PALIKA ARCHANA ET AL: "An antiviral trap made of protein nanofibrils and iron oxyhydroxide nanoparticles", NATURE NANOTECHNOLOGY, NATURE PUB. GROUP, INC, LONDON, vol. 16, no. 8, 3 June 2021 (2021-06-03), pages 918-925, XP037537017, ISSN: 1748-3387, DOI: 10.1038/S41565-021-00920-5 [retrieved on 2021-06-03] * the whole document * | 1-15 | INV. A61K33/26 A61P31/14 |
| Y | NIETO-JUAREZ JESSICA I. ET AL: "Virus removal and inactivation by iron (hydr)oxide-mediated Fenton-like processes under sunlight and in the dark", PHOTOCHEMICAL & PHOTOBIOLOGICAL SCIENCES, vol. 12, no. 9, 1 January 2013 (2013-01-01), page 1596, XP055910649, GB ISSN: 1474-905X, DOI: 10.1039/c3pp25314g Retrieved from the Internet: URL:https://pubs.rsc.org/en/content/articlepdf/2013/pp/c3pp25314g> * the whole document * | 1-15 | |

TECHNICAL FIELDS SEARCHED (IPC)

A61K
A61P

-/--

The present search report has been drawn up for all claims

| Place of search | Date of completion of the search | Examiner |
|---|---|---|
| Munich | 8 April 2022 | Houyvet-Landriscina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 4**

**EUROPEAN SEARCH REPORT**

Application Number

EP 21 38 2974

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | Abo-Zeid Yasmin ET AL: "A molecular docking study repurposes FDA approved iron oxide nanoparticles to treat and control COVID-19 infection", Eur J Pharm Sci. 153:105465., 12 July 2020 (2020-07-12), XP055854500, DOI: 10.1016/j.ejps.2020.105465 Retrieved from the Internet: URL:https://www.ncbi.nlm.nih.gov/pmc/artic les/PMC7354764/pdf/main.pdf [retrieved on 2021-10-25] * the whole document * ----- | 1-15 | |
| Y | GHARPURE SAEE ET AL: "Use of nanotechnology in combating coronavirus", 3 BIOTECH, vol. 11, no. 7, 1 July 2021 (2021-07-01), page 358, XP055910671, DE ISSN: 2190-572X, DOI: 10.1007/s13205-021-02905-6 Retrieved from the Internet: URL:https://link.springer.com/content/pdf/ 10.1007/s13205-021-02905-6.pdf> * page 11 * ----- | 1-15 | |

TECHNICAL FIELDS SEARCHED (IPC)

-/--

The present search report has been drawn up for all claims

| Place of search | Date of completion of the search | Examiner |
|---|---|---|
| Munich | 8 April 2022 | Houyvet-Landriscina |

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | ZHAO KANG ET AL: "Serum Iron Level as a Potential Predictor of Coronavirus Disease 2019 Severity and Mortality: A Retrospective Study", OPEN FORUM INFECTIOUS DISEASES, vol. 7, no. 7, 1 July 2020 (2020-07-01), XP055910667, DOI: 10.1093/ofid/ofaa250 Retrieved from the Internet: URL:https://watermark.silverchair.com/ofaa 250.pdf?token=AQECAHi208BE49Ooan9kkhW_Ercy 7Dm3ZL_9Cf3qfKAc485ysgAAAtkwggLVBgkqhkiG9w 0BBwagggLGMIICwgIBADCCArsGCSqGSIb3DQEHATAe BglghkgBZQMEAS4wEQQM_Rn7a4xN8Itn1EACAgEQgI ICjC882lshsKHYpuTcuSNkoYTLFaMhxj6VFPOpBhO1 VtnO51pMRrbHa3OenFoGn07yJVFauz1R6C7JMUG8oV zBJvzRkR6e> * abstract * ----- | 1-15 | |
| Y,D | SONNWEBER THOMAS ET AL: "Persisting alterations of iron homeostasis in COVID-19 are associated with non-resolving lung pathologies and poor patients' performance: a prospective observational cohort study", RESPIRATORY RESEARCH, vol. 21, no. 1, 21 October 2020 (2020-10-21), XP055910669, DOI: 10.1186/s12931-020-01546-2 Retrieved from the Internet: URL:https://respiratory-research.biomedcen tral.com/track/pdf/10.1186/s12931-020-0154 6-2.pdf> * abstract * ----- -/-- | 1-15 | TECHNICAL FIELDS SEARCHED (IPC) |

The present search report has been drawn up for all claims

| Place of search | Date of completion of the search | Examiner |
|---|---|---|
| Munich | 8 April 2022 | Houyvet-Landriscina |

EPO FORM 1503 03.82 (P04C01)

| | Europäisches Patentamt |
|---|---|
| | European Patent Office |
| | Office européen des brevets |

**EUROPEAN SEARCH REPORT**

Application Number

**EP 21 38 2974**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YEE JERRY ET AL: "Iron sucrose: The oldest iron therapy becomes new", AMERICAN JOURNAL OF KIDNEY DISEASES, vol. 40, no. 6, 2002, pages 1111-1121, XP028834631, ISSN: 0272-6386, DOI: 10.1053/AJKD.2002.36853 * the whole document * | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 8 April 2022 | Houyvet-Landriscina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 4 of 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. J. GUZIK et al.** *Cardiovasc Res,* 2020, vol. 116 (10), 1666-1687
- **C. LADECOLA et al.** *Cell,* 2020, vol. 183 (1), 16-27
- **PORTILLA et al.** *ACS Appl. Mater. Interfaces,* 2021, vol. 13 (7), 7924-7944
- **KUMAR et al.** *J. Infect. Chemother,* 2019, vol. 25 (5), 325-329
- **KIM et al.** *Small,* 2017, vol. 13 (32), 1700818
- **WANG et al.** *Cell Discovery,* 2018, vol. 4 (14
- **Y. ABO-ZEID et al.** *Eur. J. Pharm. Sci.,* 2020, vol. 153, 105465
- **Y. PAN.** *Small,* 2007, vol. 3, 1941-1949
- **ZHAO et al.** *Open Forum Infectious Diseases,* 2020, vol. 7 (7

- **SONNWEBER et al.** *Respiratory Research. W,* 2020, vol. 21 (1), 276
- **L. GUTIÉRREZ et al.** *Journal of Magnetism and Magnetic Materials,* 2005, vol. 293 (1), 69-74
- **T. TOPTAN et al.** *Int. J. Mol. Sci.,* 2020, vol. 21 (12), 4396
- **ALEXANDERSEN et al.** *Nature Communications,* 2020, 6059
- **K. J. LIVAK et al.** *Methods,* 2001, vol. 25 (4), 402-408
- **MULENS-ARIAS et al.** *Biomaterials,* 2015, vol. 52, 494-506
- **KHAN et al.** *Biomaterials,* 2012, vol. 33 (5), 1477-88